# EUROPEAN PATENT SPECIFICATION

(11) **EP 4 470 526 B1**
(45) Date of publication and mention of the grant of the patent: **05.08.2026**
(21) Application number: 24189318.9
(22) Date of filing: 18.07.2024
(51) Int. Cl.: A61K 9/00, A61K 9/06, A61K 31/00, A61K 47/10, A61K 47/18, A61K 47/32, A61P 31/10

(54) **TOPICAL COMPOSITION FOR ADMINISTRATION TO NAILS**
TOPISCHE ZUSAMMENSETZUNG ZUR VERABREICHUNG AN NÄGEL
COMPOSITION TOPIQUE POUR ADMINISTRATION AUX ONGLES

(30) Priority: 25.07.2023 EP 23187521
(43) Date of publication of application: 04.12.2024
(73) Proprietor: MiM Pharma GmbH, 63263 Neu-Isenburg (DE)
(72) Inventor: Herbig, Michael, 22525 Hamburg (DE); Köllmer, Melanie, 22525 Hamburg (DE); Böhm, Philipp, 22525 Hamburg (DE)
(74) Representative: Synergy IP Group AG

(56) References cited:
- DE-U1- 202008 011 653
- TANG CHUN FUNG ET AL: "Characterization of piroctone olamine for topical delivery to the skin", vol. 45, no. 3, 27 March 2023 (2023-03-27), NL, pages 345 - 353, XP093112334, ISSN: 0142-5463, Retrieved from the Internet <URL:https://onlinelibrary.wiley.com/doi/pdf/10.1111/ics.12839> [retrieved on 20231214], DOI: 10.1111/ics.12839
- PURNIMA KATARIA ET AL: "Emergence of nail lacquers as potential transungual delivery system in the management of onchomycosis", EXPERT OPINION ON DRUG DELIVERY, vol. 13, no. 7, 2 July 2016 (2016-07-02), GB, pages 937 - 952, XP055467419, ISSN: 1742-5247, DOI: 10.1080/17425247.2016.1174691

## Description

### FIELD OF THE INVENTION

The present invention relates to topical compositions that are useful for being administered to the skin or to fingernails or toenails of subjects affected with onychomycosis, also referred to as tinea unguium.

### BACKGROUND OF THE INVENTION

Onychomycosis is a rather common condition defined as a fungal infection of finger- or toenails potentially causing discolouration, thickening, and separation from the nail bed. It occurs in about 10 % of the overall population. Its prevalence increases with age to about 20 % in adults older than 60 years and about 50 % in those older than 70 years.

A nail with onychomycosis, i.e., a mycotic nail, may appear thicker and more fragile than a healthy nail, and often its colour is more brownish, whitish or even blackish than unaffected nails. If left untreated, the skin underneath and around the nail can become inflamed and painful. In some cases, an affected nail may separate from the nail bed.

Various subtypes of onychomycosis are known, including distal or lateral subungual onychomycosis, proximal subungual onychomycosis, superficial onychomycosis, edonyx onychomycosis, dystrophic onychomycosis, yeast onychomycosis, and fungal melanonychia. The pathogens causing onychomycoses are generally fungi, such as dermatophytes, yeasts, or molds. Common dermatophytes include *Trichophyton rubrum, T, violaceum, T. tonsurans, T. soudanense, T, interdigitale, Epidermophyton floccosum,* and *Microsporum gypseum.* Molds known to infect human nails include various *Scytalidium* or *Neoscytalidium, Scopulariopsis,* and *Aspergillus* species. The most common yeast infections of nails are caused by various *Candida* species such as *Candida albicans.*

Antifungal medications are available both for systemic and local treatment. Systemic therapies include oral administration of terbinafine, itraconazole, or fluconazole. These compounds may also be administered topically. Moreover, some further antifungal agents are primarily used as topical therapies primarily because they are not effective (e.g., poorly bioavailable) or not well tolerated when given by mouth. Among these are efinaconazole, ketoconazole, ciclopirox, and amorolfine.

Another compound of interest is piroctone olamine, sometimes also referred to as piroctone ethanolamine or Octopirox^{®}. Piroctone olamine exhibits some antifungal as well as antibacterial effect. It is used, for example, in medicated shampoos or other liquid compositions for use on the scalp, in particular for treating dandruff, or *Pityriasis capitis.* The compound is considered mildly effective and well tolerated, which makes it suitable also for preparations that may be developed as cosmetics rather than compositions regulated as drug products.

In general, it is rather challenging to effectively treat an infected nail with a topically administered active ingredient. This is due to the thickness, dense structure, and highly keratinised composition of the nail plate, i.e., factors which together lead to a low overall permeability for drug substances that often require repeated administration over long periods of time. Another contributing factor is the low local residence time of a topically administered conventional drug formulation such as a liquid or an ointment It has been proposed to address the permeability challenges by using formulations that incorporate keratolytic permeation enhancers such as urea or salicylic acid. This formulation strategy has been successful with some drug substances such as terbinafine. For example, WO2013/171159 discloses aqueous terbinafine compositions comprising 15 wt% to 35 wt.% of urea that show good penetration into and permeation through nails.

However, this formulation strategy based on the use of urea as keratolytic permeation enhancer is not easily applied to compositions with piroctone olamine as antifungal agent. On the one hand, urea requires an acidic environment in order to have acceptable chemical stability and to avoid the release of free ammonia over time which would cause an unpleasant smell and an increase of pressure in the primary package. On the other hand, piroctone olamine is not sufficiently soluble at such acidic pH. The addition of large amounts (e.g., 50 wt% or higher) of ethanol, or similar volatile solvents, would overcome the solubility problem of piroctone olamine but would also lead to (i) poor acceptability due to the pronounced irritation potential of such ethanol concentrations, as well as (ii) to reduced urea solubility, thus, limiting the amount of urea that can be incorporated. This can be seen, for instance, with prior art compositions such as described in DE202008011653U1 to Medicos Kosmetik GmbH: while acknowledging the keratinolytic properties of urea when used in concentrations of 10 % (w/v) or more, the authors of DE202008011653U1 can incorporate only smaller amounts well below these 10 % (w/v) in their nail polish formulations (e.g., only 1.5 % (w/v) in the exemplary nail polish of paragraph [0043]).

In addition, larger amounts of volatile solvents like ethanol, as commonly found in nail polish, for instance, also entail the risk of precipitation of dissolved urea and/or piroctone olamine upon evaporation of said solvents; thereby, rendering both substances far less permeable for the nail. Moreover, it is difficult to increase the viscosity of such formulations as required for usability and sufficient retention on the nail plate without aggravating the stability and solubility issues.

A further technical problem is that aqueous-based formulations comprising urea are inherently associated with some degradation (to ammonia and carbon dioxide) and consequently a potential increase of pressure, which would normally require the use of primary packaging containers that are gas permeable and exhibit some elasticity, such as plastic containers rather than packaging means made of metal or glass. Yet, while plastic containers are a light-weight packaging option, the water from aqueous compositions typically permeates this plastic even more than the ammonia or carbon dioxide, thus risking water losses during storage and consequently formulation changes (e.g., viscosity, concentration/ solubility of actives, etc.)

It is therefore an object of the present invention to provide an improved composition of piroctone olamine comprising urea as keratolytic permeation enhancer which is useful for administration to finger- and/or toenails in order to treat or prevent onychomycosis. A further object is to address or overcome one or more of the problems associated with the prior art formulations that have been proposed for treating onychomycosis or for the cosmetic care of nails that are or were infected with a fungus. Further objects become clear on the basis of the patent claims and the description below, including the examples.

### SUMMARY OF THE INVENTION

In a first aspect, the invention provides a water-free composition for topical administration comprising 0.1 wt.% to 1 wt.% piroctone olamine; 10 wt% to 30 wt.% urea; a hydrophilic solvent; and a polymeric viscosity-enhancing agent. The composition is in the form of a gel, and comprises not more than 20 wt% of volatile solvents.

The inventors have found that, surprisingly, a viscous, hydrophilic but at the same time non-aqueous formulation design overcomes the solubility and stability issues arising from the combination of piroctone olamine and urea. The composition has a commercially viable shelf life, good usability and adherence to the site of administration, can be easily washed off, and is expected to show high local tolerability. It is particularly useful in treating onychomycosis, or for nail care and treatment in onychomycosis, or in the prevention or treatment of conditions related to onychomycosis such as brittle nails, superficial white onychomycosis, and may be helpful in long-term prophylaxis.

As noted above, the composition is formulated as a gel. The hydrophilic solvent(s) required for a non-aqueous gel may, for example, be selected from glycerol, propylene glycol, ethanol (anhydrous), liquid polyethylene glycols, or any combinations thereof; however, in total, the composition should exhibit not more than 20 wt% of volatile solvents, such as ethanol. The composition may comprise one or more further active ingredients, for example panthenol.

In a further aspect, the invention provides a metal ointment tube comprising a gel composition as disclosed above, in particular a composition essentially consisting of about 0.25 wt% to 1 wt% piroctone olamine; about 10 wt% to 20 wt% urea; about 1 wt% to 3 wt% dexpanthenol; about 40 wt% to 65 wt% glycerol; about 10 wt% to 25 wt% propylene glycol; about 5 wt% to 15 wt% ethanol (anhydrous); about 0.25 wt% to 1 wt.% polyacrylate crosspolymer-6; and up to about 10 wt.% of one or more further active or inactive ingredients. Such product is enabled by the superior stability of the gel composition which permits the use of a cost-effective metal tube as primary package.

In a yet further aspect, the invention provides the use of the composition as defined herein in the prevention or treatment of onychomycosis or a condition related to onychomycosis.

### DETAILED DESCRIPTION OF THE INVENTION

In a first aspect, the invention provides a composition for topical administration which is characterised in that it is water-free and comprises the following constituents:
- from 0.1 wt.% to 1 wt.% piroctone olamine;
- from 10 wt% to 30 wt% urea;
- a hydrophilic solvent; and
- a polymeric viscosity-enhancing agent, wherein the composition is in the form of a gel, and comprises not more than 20 wt.% of volatile solvents.

As mentioned, it was unexpectedly found by the inventors that a viscous, non-aqueous formulation design addresses and reconciles the solubility and stability problems that arise when combining piroctone olamine and urea within the same formulation. The composition was found to be highly stable, both in terms of chemical and physical stability. Moreover, it shows a high degree of usability and patient-friendliness in that it can easily be administered, exhibits a high degree of adherence and local retention after administration to nail plates, and can be readily washed off if needed. Advantageously, both piroctone olamine and urea were found to be present in dissolved form, and no precipitation of either of these two active ingredients was observed visually within the first hour after application, or thereafter, indicating that both substances remain present in their dissolved form, which is known to permeate the nail most effectively. Moreover, it is expected to show high skin tolerability and may further contain nurturing and healing-promoting compounds such as panthenol, which is relevant for such compositions as administration to the nails typically involves some contact of the formulation with the skin as well.

The composition provided according to this aspect of the invention is explained in more detail below, along with some of the optional features and certain current preferences for carrying out this aspect of the invention.

The composition is suitable and adapted for topical administration. In particular, it is formulated and processed such as to be suitable for the administration to a fingernail or toenail of a subject such as a human subject. This implies that the constituents of the composition are selected from compounds and materials that are known to be safe for topical administration and acceptable under pharmaceutical or cosmetic regulations. Depending on its primary purpose, the composition may therefore be referred to as a pharmaceutical composition and/or as a cosmetic composition, as the case may be. Technically, there may be little or no difference between these two categories as the respective pharmaceutical and cosmetic regulations may differ some what between the various jurisdictions such that, in some cases, a particular product may represent a pharmaceutical product in one jurisdiction and a cosmetic product in another. For example, urea concentrations of 10-15 wt.% are typically considered suitable for cosmetic products in many jurisdictions, whereas higher values of e.g., 20 wt.% or 30 wt.% are often restricted to pharmaceutical compositions (with urea then being labelled as an active ingredient rather than an excipient).

Piroctone olamine is the ethanolamine salt of the hydroxamic acid derivative piroctone. Another chemical name of the compound is 1-hydroxy-4-methyl-6-(2,4,4-trimethylpentyl)-2(1H)-pyridone monoethanolamine salt. Its chemical formula is C₁₆H₃₀N₂O₃, and its molar mass is 298.421. Piroctone olamine was originally sold with the tradename of Octopirox^{®} as a component of an external formula for dandruff. In the context of the present invention, piroctone olamine is used in view of its antifungal activity. The compound is comprised in the composition at an amount of 0.1 wt.% to 1 wt.%. In some of the preferred embodiments, the composition comprises piroctone olamine at an amount of about 0.25 wt.% to 1.0 wt.%, or at an amount of about 0.25 wt% to about 0.75 wt.%. In a specific embodiment, the composition comprises piroctone olamine at an amount of about 0.50 wt.%. In this context, the expression "about" should be understood as including the tolerances that are generally accepted in the applicable technical field, such as ± 10 %, with the percentage being understood as relative to the upper or lower limit of the range. Even without specifically mentioning "about", any amount specified herein should be interpreted accordingly. In other words, the above range may also be understood as covering amounts from 0.09 wt% to 1.1 wt.%. This general principle is also applicable to other quantities described herein, as is common practice in the field.

Water-free, as used herein, means that the composition is free of technically relevant amounts of water. For example, no water is added when preparing the composition, and the constituents used for its preparation are selected as essentially water-free grades. Minor amounts of residual water may however be present in view of the typical purity of the constituents used for the product. For example, it is known that pharmaceutical grades of certain hydrophilic solvents such as ethanol may be labeled as anhydrous but in fact may contain some minor amounts of water, such as less than about 0.5 % (v/v), in that most major pharmacopoeias require that anhydrous ethanol contains at least 99.5 % (v/v) of ethanol at 20 °C. Thus, when referring to ethanol herein, this shall refer to anhydrous ethanol unless where explicitly stated otherwise. Similarly, solid excipients such as certain polymeric viscosity-enhancing agents may contain a minor amount of residual moisture. Moreover, it should be taken into account that also during processing and storage, some constituents or the composition itself may absorb water from the air, depending on the degree of hygroscopicity of the composition or the respective ingredients. For example, glycerol which may be used as a hydrophilic solvent as described below, is a rather hygroscopic compound and its incorporation may result in the absorption of minor amounts of water. In general, the overall water-content of the water-free formulation should be less than about 3 wt%. Preferably, not more than 2 wt% of water should be present. In further embodiments, the composition comprises not more than about 1.5 wt% or even 1 wt% of water, respectively. These preferences should be understood as generally applicable in the context of the invention, and combinable with all other optional features and preferences.

Urea is an organic compound with chemical formula CO(NH₂)₂, and also known as carbonyl diamide or carbamide. The molecule plays important roles in the metabolism of humans and other animals. In the context of the present invention, it is used as a key constituent of the composition based on its keratolytic activity by which it is able to soften nails and make them more permeable to topically administered active agents, such as piroctone olamine. Urea is therefore sometimes referred to as keratolytic permeation enhancer. Urea is comprised in the composition at an amount of 10 wt% to 30 wt.%. In some of the preferred embodiments, the composition comprises urea at an amount of 10 wt% to about 25 wt.%, or at an amount of 10 wt.% to about 20 wt.%, or at an amount of 10 wt% to about 15 wt.%. In some of the preferred embodiments, the composition comprises urea at an amount of about 15 wt.%. As mentioned above, urea concentrations in the range of about 10-15 wt.% are typically considered suitable for cosmetic products in many jurisdictions, with higher concentrations usually being restricted to pharmaceuticals.

The composition further comprises a hydrophilic solvent. As used herein, a solvent is a material that is liquid at normal conditions, i.e., at room temperature (20 °C to 25°C) and under standard atmospheric pressure (about 1 atm, or about 1,013.25 hPa). Hydrophilic, in this context, means that the respective material is more attracted to water than to a lipid. For a solvent, this typically implies some degree of miscibility with water. In other words, the hydrophilic solvent is a pharmaceutically and/or cosmetically acceptable liquid material that is at least partially miscible with water at normal conditions. In some preferred embodiments, the hydrophilic solvent is fully water-miscible, i.e., at any weight ratio of solvent to water.

Furthermore, the composition comprises a polymeric viscosity-enhancing agent As used herein, a viscosity-enhancing agent is a pharmaceutically and/or cosmetically acceptable material which, when incorporated in the composition in dissolved and/or dispersed form, results in an increased viscosity. Typically, a viscosity-enhancing agent has a significant impact on the viscosity of a composition, which means that even when incorporated in small amounts it substantially increases the viscosity. A viscosity-enhancing agent may also be referred to as gelling or gel-forming agent, thickener, or rheology modifier.

The viscosity-enhancing agent may represent a natural or synthetic compound or combination of compounds. In this context, polymeric means that the compound or one of the compounds forming part of the viscosity-enhancing agent is polymeric, i.e., being composed of macromolecules. A macromolecule is a molecule of high relative molecular mass, the structure of which essentially comprises the multiple repetition of units derived, actually or conceptually, from molecules of low relative molecular mass. Preferably, the average molecular mass of the macromolecules of the polymeric viscosity-enhancing agent is at least about 1,000 Dalton (Da). In further preferred embodiments, the average molecular mass is at least about 5,000 Dalton, or at least about 10,000 Dalton, respectively. In other preferred embodiments, the average molecular mass is higher than 100,000 Dalton, and optionally higher than 100,000 Dalton, or even higher than 1,000,000 Dalton, respectively.

Due to the incorporated polymeric viscosity-enhancing agent, the composition is in the form of a gel. In the context of the invention, a gel should be understood as a viscoelastic, semisolid material that is characterised by rheological properties which resemble, in part, the rheological behaviour of a viscous fluid and, also in part, that of an elastic solid. Macroscopically, a gel behaves like a solid upon the exertion of low shear force, and like a viscous fluid when the shear force exceeds a threshold which is defined as the yield point. According to a further definition, a gel is a system with a finite, usually rather small, yield stress. In one of the preferred embodiments, the composition is in the form of a gel, wherein said gel is clear and colourless.

In the context of the invention, a reference to the viscosity of a composition should be interpreted as referring to the shear viscosity, unless the context dictates otherwise. The shear viscosity, or dynamic viscosity, characterises the resistance of a material to flow or shear. The shear viscosity of topical pharmaceutical or cosmetic composition may be measured by rotational rheometry. Unless specified otherwise, the shear viscosity is determined at normal conditions as described above, in particular at room temperature, or at a temperature in the range from about 20 °C to about 25 °C.

In some preferred embodiments, the shear viscosity of the composition at a shear rate of 100 1/s is at least about 0.1 Pa*s, such as in the range from about 0.1 to about 50 Pa*s. Again, the expression "about" should be understood as accounting for the typical tolerances in the technical field as generally understood by a skilled person. In some further preferred embodiments, the shear viscosity at 100/s is in the range from about 0.1 to about 10 Pa*s. In other embodiments, shear viscosity at 100/s is from about 0.5 to about 10 Pa*s, such as about 1±0.5 Pa*s, about 2±0.5 Pa*s, about 3±0.5 Pa*s, about 4±0.5 Pa*s, or about 5±0.5 Pa*s, respectively. For the avoidance of doubt, with respect to the shear viscosity, it should be understood that the viscosity ranges described herein refer to the composition as freshly applied, i.e., to the composition as it comes out of its primary packaging. These values may increase slightly over time once applied to the nail but not by much as is shown in Example 5. As will be described in more detail further below, it is not the aim of the present invention that the composition shall harden into a solidified film on the surface of the nail after application by evaporation of a predominantly volatile solvent system (as is common with prior art nail polish formulations).

As mentioned above, a major component of the composition is the hydrophilic solvent. The advantage of the hydrophilic solvent in the water-free composition is that it brings about a user friendliness similar to that of an aqueous gel and at the same time enhances the chemical stability and the solubilisation capacity with respect to piroctone olamine and urea. Examples of potentially suitable hydrophilic solvents include glycerol, propylene glycol, ethanol, a liquid polyethylene glycol, or a combination of any of these. In this context, a liquid polyethylene glycol is a polyethylene glycol or mixture of two or more polyethylene glycol grades that is liquid at normal conditions.

The combination may, for example, be a combination of two solvents, such as glycerol and ethanol, glycerol and propylene glycol, ethanol and propylene glycol, etc.; a combination of three solvents, such as ethanol, glycerol and propylene glycol; or a combination of four solvents. In some of the preferred embodiments, the composition comprises a combination of at least two hydrophilic solvents selected from glycerol, propylene glycol, ethanol, and liquid polyethylene glycols. In some further preferred embodiments, the composition comprises a combination of atleast two hydrophilic solvents, with atleast one of them being selected from the group of glycerol, propylene glycol, and liquid polyethylene glycols. A particularly useful combination is that of the three solvents, ethanol, glycerol and propylene glycol, which is therefore used according to some further preferred embodiments.

It is noted that the expression "solvent" may be used for a single, chemically defined compound (such as ethanol) or alternatively for a liquid carrier which may represent a mixture of chemically defined compounds. Both meanings are well-established in the applicable technical field, and a person skilled in the art will understand from the context which interpretation should be applied.

The amount of the hydrophilic solvent or, in case of using a combination of chemically defined solvents, the amount of the hydrophilic solvents in the composition may preferably be selected in the range from 65 wt.% to 85 wt%, the percentage being based on the weight of the composition.

In case, one or more volatile solvent(s), such as ethanol or isopropanol, is/are used in combination with at least one further chemically defined solvent such as glycerol, propylene glycol, or liquid polyethylene glycol, it is preferred, according to some embodiments, that the volatile solvent(s) (e.g., ethanol) is/are incorporated at a level of not more than about 20 wt.%, or not more than about 15 wt.%, relative to the weight of the composition. In this context, a volatile solvent refers to solvents which vaporize, or evaporate, at normal conditions, i.e., at room temperature (20 °C to 25°C) and under standard atmospheric pressure (about 1 atm, or about 1,013.25 hPa). In some preferred embodiments, volatile solvents may be used at an amount in the range of from about 5 wt.% to about 20 wt.%, or from about 5 wt.% to about 15 wt.%. For example, when ethanol is used as a volatile solvent, ethanol may be used at an amount in the range from about 5 wt.% to about 20 wt.%. Within this range, the ethanol contributes to the solubilisation of piroctone olamine without causing skin irritation or reducing urea solubility. Also preferred is an ethanol amount in the range from about 5 wt.% to about 15 wt.%. In a specific embodiment, the composition comprises ethanol at an amount of about 10 wt.%.

In such solvent combinations, with volatile solvent(s) included, the weight ratio of the volatile solvent(s) (e.g., of ethanol) to the further solvent (or the combined further solvents, if applicable) may, for example, be in the range from about 1:20 to about 1:3, in particular from about 1:10 to about 1:5.

One of the main purposes of limiting the use of volatile solvents like ethanol in the composition is to reduce the risk of precipitation of piroctone olamine and/or urea after application of the composition to the nail (i.e., when the solvent evaporates). Precipitation of active ingredient(s) is a phenomenon often seen with prior art nail polish formulations and is discussed to be one of the reasons why, despite the extended residence time of solidified nail polish films on the nail surface, nail polishes often fail to deliver the expected results and need to be reapplied daily. Active ingredients are known to permeate best into and through the nail when present in their dissolved form; thus, once precipitated, their permeation is hindered significantly. In contrast to nail polishes, the present composition provided in the form of a gel, does not thicken significantly when the small amounts of volatile solvent(s) contained therein evaporate, let alone hardens into a solidified film on the nail surface. Instead, the composition according to the present invention stays viscous to keep the active ingredients in their dissolved form, with the shear viscosity of the applied composition after, for instance about 1 hour (when all volatile solvent is assumed to be evaporated), being only marginally higher than the viscosity ranges described above for the composition as such, or as freshly applied from its primary packaging.

As indicated above, one further reason for limiting the use of volatile solvents like ethanol in the composition is to allow sufficient concentrations of dissolved urea, and specifically to allow the incorporation of the desired 10-30 wt.% urea in order to utilise the keratolytic properties thereof.

If glycerol is used in combination with at least one further chemically defined solvent such as ethanol, propylene glycol, or liquid polyethylene glycol, it is, according to some of the preferred embodiments, used at an amount of at least about 30 wt.%, relative to the weight of the composition. In some further embodiments, the amount of glycerol is in the range from about 30 wt.% to about 70 wt.%. In other preferred embodiments, the amount of glycerol is in the range from about 35 wt.% to about 65 wt.%, such as from about 40 wt.% to about 65 wt.%, or from about 45 wt.% to about 65 wt.%, respectively, such as about 50 wt.% or about 60 wt.%.

Propylene glycol, if used in combination with glycerol and optionally one or more further chemically defined hydrophilic solvent, may preferably be incorporated at an amount that is less than the amount of the glycerol. For example, propylene glycol may be used at an amount in the range from about 5 wt.% to about 25 wt.%, or in the range from about 10 wt.% to about 25 wt.%, relative to the weight of the composition. In further preferred embodiments, the amount of propylene glycol is in the range from about 5 wt% to about 20 wt.%, from about 5 wt% to about 15 wt.%, or from about 10 wt.% to about 20 wt.%, or from about 10 wt.% to about 15 wt.%, respectively, such as about 12 wt.%.

The liquid polyethylene glycol may, for example, be macrogol 300, macrogol 400, or a mixture of these. In some embodiments, it is used in combination with at least one other chemically defined hydrophilic solvent such as ethanol and/or glycerol and incorporated at an amount in the range from about 10 wt.% to about 35 wt.%, relative to the weight of the composition. According to some further embodiments, the liquid polyethylene glycol is macrogol 300 and incorporated at an amount from about 15 wt.% to about 30 wt.%.

In some particularly preferred embodiments, the hydrophilic solvent represents a combination of about 40 wt.% to 65 wt.% glycerol, about 10 wt.% to 25 wt% propylene glycol, and about 5 wt.% to 15 wt.% ethanol, wherein all percentages are relative to the weight of the composition. In some further preferred embodiments, the hydrophilic solvent is a combination of about 50-60 wt.% glycerol, about 10-15 wt% propylene glycol, and about 5-15 wt% ethanol, wherein all percentages are relative to the weight of the composition.

As mentioned, the composition is non-aqueous but still hydrophilic, due to the use of the hydrophilic solvents(s) as carrier. The hydrophilic nature is useful for enhancing both the user friendliness and tolerability of the product as well as the urea solubility. Accordingly, in some related embodiments, the composition is essentially free of lipids or oils, or at least free of technically relevant amounts thereof. Minute amounts of oils (such as essential oils) may, for example, be present in perfume components that may optionally be incorporated. However, such minute amounts are technically insignificant with respect to the carrier properties of the carrier based on the hydrophilic solvent(s). Also related are some preferred embodiments according to which the composition is not in the form of an emulsion (including microemulsions). In other words, the composition is preferably free of a dispersed phase consisting of oil or lipid droplets.

Moreover, as indicated above, according to preferred embodiments, the composition is not in the form of a nail polish (alternatively referred to as a nail lacquer or nail varnish). In other words, the composition is neither based on a predominantly volatile solvent system, nor aimed at hardening into a solidified film after application to the nail.

Both the absence of lipids or oils and the absence of a predominantly volatile solvent system differentiate the composition as disclosed herein from prior art compositions comprising urea and piroctone olamine, such as the nail polish as described in DE202008011653U1, which employs isatis oil and octyldodecanol as well as a solvent system that introduces over 80 % volatile solvents into the nail polish and allows for less than 5 wt.% urea content.

The polymeric viscosity-enhancing agent, which was already defined above in generic terms, may be selected from any pharmaceutically or cosmetically acceptable polymeric excipients with high viscosity that are in principle compatible with hydrophilic solvents even in the absence of water. Examples of viscosity-enhancing agents that have been shown to function as gel-forming agents in water-free systems include various acrylic acid-based polymers such as carbomers, xanthan gum, certain cellulose ethers such as hydroxyethylcellulose. Moreover, hydrophilic gels incorporating polymeric viscosity-enhancing agents have been described that are not in all jurisdictions currently accepted for topical human use by regulatory agencies, such as poly(monostearoyl glycerol-co-succinate) polymer. Once such excipients are generally considered acceptable in topical compositions, these may also be considered as potentially useful.

The inventors have currently achieved best results with viscosity-enhancing agents selected from the class of acrylic acid-based polymers, copolymers or crosspolymers. Accordingly, in some preferred embodiments, the viscosity-enhancing agent is selected from this class. As used herein, acrylic acid-based polymers are polymers comprising repeating optionally substituted acrylic acid units. Moreover, a copolymer should be understood as a polymer derived from more than one species of monomer. Depending on how the repeating units are arrange within the macromolecules, various types of copolymers may be distinguished, such as alternating copolymers, random copolymers, block copolymers, and graft copolymers. A crosspolymer, as understood in the context of the invention is a type of polymer that is formed by crosslinking monomer units in a network structure. Crosslinking refers to the formation of chemical or strong physical bonds between the polymer chains or monomers that make up the material, creating a three-dimensional network structure.

Examples of potentially useful acrylic acid-based homopolymers include, without limitation, poly(acrylic acid), polymethyl methacrylate (PMMA), poly(ethyl methacrylate) (PEMA), or poly(2-hydroxyethyl methacrylate) (pHEMA). Acrylic acid-based copolymers include, for example, methacrylic acid-ethyl acrylate copolymers, also referred to as poly(ethyl acrylate, methyl methacrylate). Crosslinked acrylic acid-based polymers include, for example, carbomers, which are high-molecular-weight polymers of acrylic acid that are crosslinked with either allyl sucrose or allyl ethers of pentaerythritol; polycarbophil, a high molecular weight acrylic acid polymer crosslinked with divinyl glycol.

Potentially useful acrylic acid-based crosspolymers are also chemically crosslinked and most of the represent copolymers at the same time. Such crosspolymers include, for example, methyl methacrylate crosspolymer, a copolymer of methyl methacrylate crosslinked with glycol dimethacrylate; methyl methacrylate/glycol dimethacrylate crosspolymer, a cross-linked copolymer of methyl methacrylate and ethylene glycol dimethacrylate monomers; polyacrylate crosspolymer-6, which is a copolymer of ammonium acryloyldimethyltaurate, dimethylacrylamide, lauryl methacrylate and laureth-4 methacrylate, crosslinked with trimethylolpropane triacrylate; acrylates/C₁₀₋₃₀ alkyl acrylate crosspolymer; acrylates/C₁₂₋₁₃ alkyl methacrylates/methoxyethyl acrylate crosspolymer; acrylates crosspolymer; acrylates/ethylhexyl acrylate crosspolymer; acrylates/ethylhexyl acrylate/glycidyl methacrylate crosspolymer; acrylates/PEG-4 dimethacrylate crosspolymer; Acrylates/Steareth-20 methacrylate crosspolymer; acrylates/vinyl isodecanoate crosspolymer; acrylates/vinyl neodecanoate crosspolymer; allyl methacrylate/glycol dimethacrylate crosspolymer; allyl methacrylates crosspolymer; butyl acrylate/glycol dimethacrylate crosspolymer; C₈₋₂₂ alkyl acrylates/methacrylic acid crosspolymer; glycol dimethacrylate/vinyl alcohol crosspolymer; lauryl methacrylate/glycol dimethacrylate crosspolymer; lauryl methacrylate/sodium methacrylate crosspolymer; methacrylic acid/PEG-6 methacrylate/PEG-6 dimethacrylate crosspolymer; PEG/PPG-5/2 methacrylate/methacrylic acid crosspolymer; potassium acrylates/C₁₀₋₃₀ alkyl acrylate crosspolymer; sodium acrylates crosspolymer-2; sodium acrylates/C₁₀₋₃₀ alkyl acrylate crosspolymer; sodium acrylates/vinyl isodecanoate crosspolymer; stearyl/lauryl methacrylate crosspolymer.

According to some preferred embodiments, the polymeric viscosity-enhancing agent is selected from crosslinked polyacrylate-based polymers including in particular carbomers and acrylic acid-based crosspolymers. In further preferred embodiments, an acrylic acid-based crosspolymer is used. Further preferred is the use of polyacrylate crosspolymer-6 as viscosity-enhancing agent. An example of a commercially available grade of a polyacrylate crosspolymer-6 is known under the commercial name SEPIMAX ZEN^{™} (Seppic).

The inventors have found that this excipient readily forms a very homogeneous gel in the absence of water, in particular when a combination of two or more members of the group consisting of glycerol, propylene glycol, ethanol, a liquid polyethylene glycol is selected as carrier, or hydrophilic solvents, to carry out this aspect of the invention.

Accordingly, one preferred embodiment provides a water-free composition for topical administration comprising 0.1 wt% to 1 wt% piroctone olamine; 10 wt.% to 30 wt.% urea; at least two hydrophilic solvents selected from glycerol, propylene glycol, ethanol, and liquid polyethylene glycols; and polyacrylate crosspolymer-6 as polymeric viscosity-enhancing agent.

In some further embodiments, the amount of the polyacrylate-based polymer, copolymer or crosspolymer in the composition is about 0.1 wt% to 1 wt.%, relative to the weight of the composition. This range also applies in the case that a carbomer an acrylic acid-based crosspolymer is used in the composition to function as the viscosity-enhancing agent. In a further preferred embodiment, the composition comprises from about 0.25 wt.% to about 1 wt% polyacrylate crosspolymer-6, such as 0.25±0.1 wt.%, 0.5±0.25 wt.%, 0.75±0.25 wt.%, or 1.0±0.25 wt.%, respectively. For example, the water-free composition may comprise about 0.25 wt.% to 1 wt% piroctone olamine; 10 wt% to 20 wt% urea; about 40 wt.% to 65 wt.% glycerol; about 10 wt% to 25 wt% propylene glycol; about 5 wt% to 15 wt% ethanol; about 0.25 wt.% to 1 wt% polyacrylate crosspolymer-6; and up to about 10 wt% of one or more further active or inactive ingredients. In some preferred embodiments, the water-free composition may comprise about 0.25 wt.% to 0.75 wt.% piroctone olamine; 10 wt% to 15 wt% urea; about 50-60 wt% glycerol, about 10-15 wt% propylene glycol, and about 5-15 wt.% ethanol; about 0.25 wt.% to 0.75 wt.% polyacrylate crosspolymer-6; and up to about 10 wt% of one or more further active or inactive ingredients, wherein all percentages are relative to the weight of the composition.

As said, one or more further ingredients may be incorporated in the composition without departing from the inventive concept. Optionally, a further active ingredient may be incorporated. Keeping in mind the main purpose of the composition, compounds that improve the healing of damaged tissue such as skin are examples of potentially useful further active ingredients. In some embodiments, the composition comprises dexpanthenol, also referred to as panthenol or pantothenol. Dexpanthenol is an alcohol analogue of pantothenic acid (vitamin B5) and is thus a provitamin of B5. The compound may also be used in the composition for its moisturising effect. In other words, the ingredient may be considered an active or inactive ingredient, depending on regulatory aspects in a particular jurisdiction and on the primary purpose of its incorporation.

Optionally, dexpanthenol may, for example, be used in the composition at an amount of about 0.1 to about 10 wt.%. According to some further preferred embodiments, the amount of the dexpanthenol in the composition is about 1 wt.% to about 5 wt.%, relative to the weight of the composition. In further preferred embodiments, the amount of dexpanthenol is in the range from about 1 wt.% to about 3 wt.%, or at an amount of 1.0±0.5 wt.%, 1.5±0.5 wt.%, 2.0±0.5 wt.%, 2.5±0.5 wt.%, or 3.0±0.5 wt.%, respectively.

One or more further active or inactive ingredients may be present Preferably, the total amount of the one or more further active and/or inactive ingredients is not more than about 20 wt% relative to the weight of the composition. In some further preferred embodiments, the one or more further active and/or inactive ingredients are present at an amount of not more than about 10 wt.% in the composition, or, in other words, up to about 10 wt.%. For the avoidance of doubt, this also means that these further active or inactive ingredients are optional, and that these constituents may be present at an amount of 0 wt%. According to some further preferred embodiments, the composition comprises no further active ingredient having antifungal activity; or the composition may be free of any further active ingredient.

In some specific embodiments, the composition essentially consists of:
- 0.25 wt.% to 1 wt% piroctone olamine;
- 10 wt.% to 20 wt.% urea;
- 1 wt.% to 3 wt% dexpanthenol;
- 40 wt.% to 65 wt.% glycerol;
- 10 wt.% to 25 wt.% propylene glycol;
- 5 wt% to 15 wt% ethanol;
- 0.25 wt.% to 1 wt% polyacrylate crosspolymer-6; and
- up to 10 wt.% of one or more further active or inactive ingredients

One of the particular advantages of overcoming the stability challenges resulting from the combination of piroctone olamine and urea according to an aspect of the invention is the avoidance of gas generation or a potential buildup of pressure within the primary packaging container during storage. This allows the use of cost-effective containers such as metal (e.g., aluminum) ointment tubes which are essentially impermeable to gases. In principle, the composition provided according to the disclosure herein may be packaged in any suitable container for topically administered viscous liquids or viscoelastic gels, such as squeezable plastic bottles or tubes made of e.g., polypropylene or the like, or non-squeezable plastic, glass or metal containers, optionally comprising a dispensing pump, or metal ointment tubes, in particular aluminum tubes. The tubes may optionally be coated internally, such as with polytetrafluoroethylene (PTFE) or hydrophobic coating materials to prevent or reduce the adhesion of the composition to the aluminum.

In some preferred embodiments, the composition as described above, i.e., with any of its optionally or preferred features, is provided in a metal ointment tube. Therefore, according to an aspect of the invention, a metal ointment tube is provided which contains a water-free composition for topical administration comprising 0.1 wt% to 1 wt% piroctone olamine; 10 wt% to 30 wt% urea; a hydrophilic solvent; and a polymeric viscosity-enhancing agent, and wherein the composition is in form of a gel. Preferably, the metal is or comprises aluminum. It should be understood that the preferred and optional features and feature combinations described above in the context of the composition as such also apply to the metal ointment tube comprises such composition.

According to certain specific embodiments, the metal tube comprises a composition essentially consisting of
- 0.25 wt.% to 1 wt.% piroctone olamine;
- 10 wt.% to 20 wt% urea;
- 1 wt.% to 3 wt% dexpanthenol;
- 40 wt.% to 65 wt.% glycerol;
- 10 wt.% to 25 wt.% propylene glycol;
- 5 wt.% to 15 wt.% ethanol;
- 0.25 wt.% to 1 wt.% polyacrylate crosspolymer-6; and
- up to 10 wt.% of one or more further active or inactive ingredients.

A further aspect of the invention and of the disclosure herein relates to the use of the composition according to any of the embodiments described above. In particular, it relates to the use for topical administration to the skin or to one or more nails of a subject, such as to one or more fingernails or toenails of a subject in need thereof, preferably a human subject. In some embodiments, the composition is used for the prevention or treatment of a superficial fungal infection or of a condition related to a superficial fungal infection, such as a fungal infection of the skin or of one or more nails. In some preferred embodiments, the composition as described above is used in the prevention or treatment of onychomycosis or of a condition related to onychomycosis. This is regardless of whether the use is, from a drug regulatory perspective, a cosmetic or therapeutic use.

The treatment of onychomycosis preferably involves the topical administration of the composition to one or more finger- or toenails infected with a fungus such as a dermatophyte (such as *Trichophyton rubrum, T. violaceum, T. tonsurans, T. soudanense, T. interdigitale, Epidermophyton floccosum,* or *Microsporum gypseum*), a yeast (such as from the *Candida* genus, e.g., *Candida albicans*)*,* or a mold (such as a species of *Scytalidium, Neoscytalidium, Scopulariopsis,* or *Aspergillus*). Moreover, the onychomycosis may be of any type, including without limitation distal and lateral subungual onychomycosis, proximal subungual onychomycosis, superficial onychomycosis, edonyx onychomycosis, and dystrophic onychomycosis. The treatment may be conducted in combination with one or more other oral or topical drug therapies, such as with terbinafine, itraconazole, fluconazole, efinaconazole, ketoconazole, ciclopirox, or amorolfine.

The treatment may be conducted over a period of several days, weeks or months. In some preferred embodiments, it is conducted at least once daily over a period of at least one week, or at least two weeks, respectively. In some embodiments, the treatment involves topical administration of the composition at least twice a day.

The use of the composition in the prevention of onychomycosis may, for example, be conducted in order to prevent the re-infection of nails affected by onychomycosis after successful treatment leading to the eradication of the fungus. Other preventive uses include the administration of the composition in situations in which a subject experiences an increased risk of becoming infected with onychomycosis, such as when doing sports or physical exercised in larger groups or when using public facilities or the like.

Conditions relating to onychomycosis include, for example, symptoms that arise from onychomycosis which persist even after the eradication of the fungus, such as brittle or deformed nails.

It should be understood that the uses described above inherently also involve a method of treating a subject affected by onychomycosis or a condition relating to onychomycosis, said method comprising a step of topically administering the composition according any of the embodiments described herein to a nail of the subject. Also inherent is the use of such composition in the manufacture of a medicament for the treatment of onychomycosis or a condition relating to onychomycosis.

### EXAMPLES

### Example 1: Preparation of Compositions 1.1 to 1.4

The compositions specified in Table 1 were prepared as follows, using measured amounts of the respective ingredients. Piroctone olamine was added to the ethanol and dissolved under stirring at room temperature. Continuing stirring, the polymeric viscosity-enhancing agent was added to the solution and homogeneously dispersed, resulting in component A.

In a separate vessel, the prescribed amount of propylene glycol was mixed with the glycerol, and the dexpanthenol was added to the mixture under stirring at about 50 °C. Upon achieving a clear solution, the urea was added while continuing the stirring at about 50°C until dissolved. The solution (component B) was then allowed to cool to room temperature.

The final compositions were then prepared by mixing the two components A and B under stirring. Compositions 1.1 and 1.2 were viscous liquids (not covered by the claimed invention), compositions 1.3 and 1.4 were gels. Samples of all compositions were filled into glass vials and stored at 5 °C, 25 °C, 40°C and at a temperature cycle alternating between -5 °C and 40 °C, with each temperature being held for 12 hours. Further samples of composition 1.3 were filled into coated aluminum ointment tubes of 20 mL nominal volume and stored at 5 °C, 25°C, 30 °C and 40 °C. For each temperature, tubes were stored in the horizontal and in the vertical (both with upward and downward oriented closure) orientation.

**Table 1**

| Composition | 1.1 | 1.2 | 1.3 | 1.4 |
|---|---|---|---|---|
| Constituents [wt.%] | | | | |
| Piroctone olamine | 0.50 | 0.50 | 0.50 | 0.50 |
| Urea | 15.00 | 15.00 | 15.00 | 15.00 |
| Dexpanthenol | 2.00 | 2.00 | 2.00 | 2.00 |
| Glycerol | 50.00 | 60.00 | 60.00 | 60.00 |
| Propylene glycol | - | 12.40 | 12.00 | 12.25 |
| Polyethylene glycol 400 | 22.15 | - | - | - |
| Ethanol | 10.00 | 10.00 | 10.00 | 10.00 |
| Polyacrylate crosspolymer-6¹ | 0.35 | 0.10 | 0.50 | 0.25 |
| Total | 100.00 | 100.00 | 100.00 | 100.00 |

| | | | | |
|---|---|---|---|---|
| ¹ SEPIMAX ZEN^{™} (Seppic) | | | | |

### Example 2: Testing of Compositions 1.1 to 1.4

Microscopy: The compositions prepared and stored in glass vials as described in Example 1 were tested after 3 weeks. No crystals were observed in any of the compositions. There was no difference in the microscopic appearance observed even after temperature cycling.

The other tests below were performed with the samples stored in tubes for 6 weeks and 12 weeks, respectively.

Shear viscosity: The initial shear viscosity at 100_{*}1/s was 1.87. After storage, the viscosities showed little change except for the sample stored for 12 weeks at 40 °C, as shown in Table 2.

**Table 2: Shear viscosity of composition 1.3 after storage**

| Storage temperature | 5 °C | 25 °C | 30 °C | 40 °C |
|---|---|---|---|---|
| Viscosity [Pa*s] | | | | |
| After 6 weeks | 1.87 | 1.86 | 1.86 | 1.85 |
| After 12 weeks | 1.70 | 1.81 | 1.77 | 1.40 |

Piroctone olamine content: The content of piroctone olamine was determined by HPLC before storage and after 6 and 12 weeks. The initial content was 0.506 wt.%. No significant difference was found between the tubes stored at the same temperatures but in different orientations. The results are therefore provided in Table 3 as means of samples from 3 tubes stored in different orientations.

**Table 3: Piroctone olamine content after storage**

| Storage temperature | 5 °C | 25 °C | 30 °C | 40 °C |
|---|---|---|---|---|
| Content in wt.% of composition | | | | |
| After 6 weeks | 0.509 | 0.505 | 0.506 | 0.503 |
| After 12 weeks | 0.503 | 0.503 | 0.501 | 0.497 |

| Content in % of initial value | | | | |
|---|---|---|---|---|
| After 6 weeks | 100.51 | 99.85 | 99.91 | 99.32 |
| After 12 weeks | 99.39 | 99.39 | 98.99 | 98.20 |

In result, the compositions were found to exhibit a very good stability, especially in view of the known and expected stability issues of combinations of piroctone olamine and urea.

### Example 3: Comparative example 1

Based on a screening of aqueous gel prototype formulations comprising piroctone olamine and urea, two promising candidate compositions (compositions 3.1 and 3.2) were prepared and tested. The ingredients and their amounts are specified in Table 4.

**Table 4**

| Constituents [wt.%] | 3.1 | 3.2 |
|---|---|---|
| Piroctone olamine | 0.50 | 0.50 |
| Urea | 20.00 | 20.00 |
| Dexpanthenol | 2.00 | 2.00 |
| Propylene glycol | 8.00 | 8.00 |
| Polysorbate 80 | 15.00 | 15.00 |
| Citric acid, anhydrous | 2.56 | - |
| L(+)-lactic acid | - | 4.00 |
| Ethanol, anhydrous | 8.00 | 8.00 |
| Xanthan gum | 0.50 | 0.50 |
| Water, deionised | 43.44 | 42.00 |
| Total | 100.00 | 100.00 |

The preparation method was similar to that described in Example 1, i.e., by mixing a first and a second component (components A and B), except that component A was prepared from the piroctone olamine, ethanol, propylene glycol, and polysorbate, and that component B was prepared from the other ingredients at about 70 °C. Samples were filled into flexible white bottles and tubes made of LDPE.

Compositions 3.1 and 3.2 result in clear, homogeneous gels with good adhesive properties on the nail plate. Yet, based on a better buffering capacity at weakly acidic pH values, composition 3.1 was selected for preliminary stability testing, which revealed significant stability issues. For example, after storage over 6 weeks at 40 °C, as well as after storage over 12 weeks at 30 °C and 40 °C, the packaging container slightly turned yellow and experienced some deformation due to internal pressure. The initially clear gel was opaque after 12 weeks at 40 °C, and it had developed a stronger aromatic odour compared to freshly prepared samples. The pH increased significantly over time, from an initial pH of below 4 to above pH 8 after 12 weeks at 40 °C. The piroctone olamine content decreased significantly over time at all temperature levels and in both types of LDPE containers. For example, after 12 weeks of storage at 25 °C, the piroctone olamine content had decreased from an initial value of 0.520 wt.% in LDPE tubes and 0.524 wt.% in LDPE bottles to 0.372 wt% in LDPE tubes and 0.387 wt.% in LDPE bottles, respectively.

### Example 4: Comparative example 2

Prior art compositions comprising piroctone olamine and urea were also found in DE202008011653U1 to Medicos Kosmetik GmbH (briefly referred to herein as DE'653), e.g., the water-free antifungal nail polish of paragraph [0043]. While addressing the keratolytic properties of urea concentrations of 10 % (w/v) and more, only 1.5 % (w/v) were actually incorporated in said nail polish.

In order to show that this significantly lower concentration is not a random selection but in fact the outcome of solubility limitations, comparative formulations 4.1 and 4.2 were prepared: 4.1 with 1.73 wt% urea and 4.2 with 10.00 wt.% urea. Formulation 4.1 equals the nail polish of DE'653 with the 1.50 % (w/v) urea suggested in [0043], and formulation 4.2 is a variation thereof with a urea amount closer to the 10 % suggested as a keratolytic in paragraph [0021] of DE202008011653U1. The ingredients and their amounts are specified in Table 5, with the different percentage values used in [0043], both (w/v) and (v/v), being converted to wt.% via the density and scaled to 100 %.

Since no specific preparation instructions were provided in paragraph [0043], the inventors proceeded to manufacture formulation 4.1 as follows:
- weighing piroctone olamine, urea, isopropanol, and ethanol, and mix until dissolved,
- weighing and adding in isatis oil, 2-octyldodecanol, and Luviskol,
- mixing the blend for 15 minutes at 40 °C,
- once visually homogenous, allowing the formulations to cool-down.

Formulation 4.2 was then manufactured by combining 91.58 wt.% of formulation 4.1 and 8.42 wt.% urea, and then mixing the blend for 1 hour at 40 °C.

Upon visual inspection, formulation 4.1 exhibited a clear, homogenous appearance and a light-yellow colour. In contrast, formulation 4.2 exhibited pronounced turbidity due to undissolved urea crystals, with a cloudy liquid and a sediment; thus, indicating that it is not possible to incorporate urea in keratolytic concentrations into the non-aqueous, oil-containing nail polish formulation of DE202008011653U1.

**Table 5**

| Constituents [wt.%] | 4.1 | 4.2 |
|---|---|---|
| Piroctone olamine | 0.58 | 0.53 |
| Isatis oil | 4.83 | 4.42 |
| Urea | 1.73 | 10.00 |
| 2-Octyldecaonol | 4.54 | 4.16 |
| Isopropanol | 19.01 | 17.41 |
| Ethanol | 8.89 | 8.14 |
| Luviskol^{®} VA37 I | 60.42 | 55.34 |
| Total | 100.00 | 100.00 |

### Example 5: Viscosity right after application vs. 1 hour later

Samples of the composition 1.3 of Example 1 were analysed in terms of viscosity right after application as well as one hour after application (i.e., measurement was started either directly after application of the formulation on the plate of the rheometer or 1 h after the application). Viscosity curves based on thirty-one measurement points were recorded at 25 °C and over a shear rate range 0.001 to 1000 1/s (logarithmic ramp) using a rotational rheometer (MCR 102, Anton Paar GmbH), and the cone-plate CP25 measuring system. Data collection and evaluation were performed with the software RheoCompass (also Anton Paar GmbH). The resting time was 5 minutes, and Evaporation protection was used during the actual measurements. Each condition was tested in duplicate (n=2).

The viscosity curves of both right after application vs. 1 hour after application (not depicted herein) showed very little difference, with both curves exhibiting a high viscosity in low shear conditions (representing the resting state of the formulation), and shear-thinning properties at higher shear conditions as is characteristic for gel compositions. The curve of the dried formulation exhibited a minimal shift to higher viscosities in low shear conditions; however, both viscosity curves approached each other under higher shear conditions, indicating that no major change in the formulation occurs after application. Also, it can be assumed that the gel composition will not run off its applied location but can be washed off. Table 6 presents the viscosity values of fresh and dried compositions in high and low shear conditions.

**Table 6**

| Condition | Test | Viscosity [mPas*s] at shear rate | |
|---|---|---|---|
| | | 0.01 1/s | 1000 1/s |
| Freshly applied | 1 | 7273.10 | 887,78 |
| | 2 | 7525.00 | 875.67 |
| | Mean | 7399.05 | 881.73 |
| 1 hour after application | 1 | 8432.50 | 891.05 |
| | 2 | 8000.10 | 896.36 |
| | Mean | 8216.30 | 893.71 |

## Claims

1. A water-free composition for topical administration comprising:
- 0.1 wt.% to 1 wt% piroctone olamine;
- 10 wt% to 30 wt% urea;
- a hydrophilic solvent; and
- a polymeric viscosity-enhancing agent,
wherein the composition is in the form of a gel, and
wherein the composition comprises not more than 20 wt% of volatile solvents.

2. The composition of claim 1, having a shear viscosity, as measured at room temperature by rotational rheometry at 100 1/s, of 0.1 to 10 Pa*s.

3. The composition of any one of the preceding claims, wherein the hydrophilic solvent is glycerol, propylene glycol, ethanol, a liquid polyethylene glycol, or a combination of any of these, optionally a combination of glycerol, propylene glycol, and ethanol.

4. The composition of any one of the preceding claims, wherein the amount of hydrophilic solvent(s) in the composition is 65-85 wt%.

5. The composition of any one of the preceding claims, wherein the composition comprises not more than 15 wt% of volatile solvents.

6. The composition of any one of the preceding claims, wherein the viscosity-enhancing agent is a polyacrylate-based polymer, copolymer or crosspolymer.

7. The composition of claim 6, wherein the amount of the polyacrylate-based polymer, copolymer or crosspolymer in the composition is 0.1 wt% to 1 wt.%.

8. The composition of claim 7, wherein the viscosity-enhancing agent is polyacrylate crosspolymer-6.

9. The composition of any one of the preceding claims, further comprising dexpanthenol.

10. The composition of claim 9, wherein the amount of the dexpanthenol in the composition is 1 wt% to 5 wt.%.

11. The composition of any one of the preceding claims, being essentially free of lipids or oils.

12. The composition of any one of the preceding claims, wherein the composition is not in the form of a nail polish.

13. A metal ointment tube comprising the composition of claim 1
wherein optionally the composition comprises:
- 0.25 wt.% to 1 wt% piroctone olamine;
- 10 wt% to 20 wt% urea;
- 40 wt.% to 65 wt.% glycerol;
- 10 wt% to 25 wt% propylene glycol;
- 5 wt.% to 15 wt.% ethanol;
- 0.25 wt.% to 1 wt.% polyacrylate crosspolymer-6; and
- up to 10 wt.% of one or more further active or inactive ingredients; and
wherein further optionally the composition essentially consists of:
- 0.25 wt% to 1 wt% piroctone olamine;
- 10 wt.% to 20 wt.% urea;
- 1 wt% to 3 wt.% dexpanthenol;
- 40 wt.% to 65 wt% glycerol;
- 10 wt.% to 25 wt% propylene glycol;
- 5 wt% to 15 wt% ethanol;
- 0.25 wt% to 1 wt% polyacrylate crosspolymer-6.

14. The composition as defined in any one of the preceding claims for use in the prevention or treatment of a superficial fungal infection or of a condition related to a superficial fungal infection, wherein optionally the superficial fungal infection is onychomycosis.

## Patentansprüche

1. Eine wasserfreie Zusammensetzung zur topischen Anwendung, umfassend:
- 0.1 Gew.% bis 1 Gew.% Pirocton-Olamin;
- 10 Gew.% bis 30 Gew.% Urea;
- ein hydrophiles Lösungsmittel; und
- ein polymeres viskositäts-verbesserndes Agens,
wobei die Zusammensetzung in Form eines Gels vorliegt, und
wobei die Zusammensetzung nicht mehr als 20 Gew.% flüchtige Lösungsmittel umfasst.

2. Die Zusammensetzung nach Anspruch 1, wobei diese eine Scherviskosität von 0.1 bis 10 Pa*s aufweist, gemessen durch Rotationsrheometrie bei Raumtemperatur und 100 1/s.

3. Die Zusammensetzung nach einem der vorhergehenden Ansprüche, wobei das hydrophile Lösungsmittel Glycerol, Propylenglykol, Ethanol, ein flüssiges Polyethylenglykol, oder eine Mischung aus jedwedem von diesen ist, optional eine Mischung aus Glycerol, Propylenglykol, und Ethanol.

4. Die Zusammensetzung nach einem der vorhergehenden Ansprüche, wobei der Gehalt des hydrophilen Lösungsmittels in der Zusammensetzung 65 Gew.% bis 85 Gew.% beträgt.

5. Die Zusammensetzung nach einem der vorhergehenden Ansprüche, wobei die Zusammensetzung nicht mehr als 15 Gew.% flüchtige Lösungsmittel umfasst.

6. Die Zusammensetzung nach einem der vorhergehenden Ansprüche, wobei das polymere viskositäts-verbessernde Agens ein Polyacrylat-basiertes Polymer, Copolymer, oder Crosspolymer ist.

7. Die Zusammensetzung nach Anspruch 6, wobei der Gehalt des Polyacrylat-basierten Polymers, Copolymer oder Crosspolymers in der Zusammensetzung 0.1 Gew.% bis 1 Gew.% beträgt.

8. Die Zusammensetzung nach Anspruch 7, wobei das polymere viskositäts-verbessernde Agens Polyacrylate Crosspolymer-6 ist.

9. Die Zusammensetzung nach einem der vorhergehenden Ansprüche, ferner umfassend Dexpanthenol.

10. Die Zusammensetzung nach Anspruch 9, wobei der Gehalt des Dexpanthenols in der Zusammensetzung 1 Gew.% bis 5 Gew.% beträgt.

11. Die Zusammensetzung nach einem der vorhergehenden Ansprüche, wobei diese im Wesentlichen frei von Lipiden oder Ölen ist.

12. Die Zusammensetzung nach einem der vorhergehenden Ansprüche, wobei die Zusammensetzung nicht in Form eines Nagellacks vorliegt.

13. Eine metallische Salbentube umfassend die Zusammensetzung Anspruch 1, wobei die Zusammensetzung optional umfasst:
- 0.25 Gew.% bis 1 Gew.% Pirocton-Olamin;
- 10 Gew.% bis 20 Gew.% Urea;
- 40 Gew.% bis 65 Gew.% Glycerol;
- 10 Gew.% bis 25 Gew.% Propylenglykol;
- 5 Gew.% bis 15 Gew.% Ethanol;
- 0.25 Gew.% bis 1 Gew.% Polyacrylate Crosspolymer-6; und
- bis zu 10 Gew.% von einem oder mehreren weiteren aktiven oder inaktiven Inhaltsstoffen; und
wobei die Zusammensetzung ferner optional im Wesentlichen besteht aus:
- 0.25 Gew.% bis 1 Gew.% Pirocton-Olamin;
- 10 Gew.% bis 20 Gew.% Urea;
- 1 Gew.% bis 3 Gew.% Dexpanthenol;
- 40 Gew.% bis 65 Gew.% Glycerol;
- 10 Gew.% bis 25 Gew.% Propylenglykol;
- 5 Gew.% bis 15 Gew.% Ethanol;
- 0.25 Gew.% bis 1 Gew.% Polyacrylate Crosspolymer-6.

14. Die Zusammensetzung nach einem der vorhergehenden Ansprüche zur Verwendung bei der Vorbeugung oder Behandlung einer oberflächlichen Pilzinfektion oder eines mit einer oberflächlichen Pilzinfektion zusammenhängenden Zustands, wobei die oberflächliche Pilzinfektion optional eine Onychomykose ist.

## Revendications

1. Composition anhydre destinée à une administration topique, comprenant :
- 0,1 % en poids à 1 % en poids de piroctone olamine ;
- 10 % en poids à 30 % en poids d'urée ;
- un solvant hydrophile ; et
- un agent polymère augmentant la viscosité,
dans laquelle la composition se présente sous la forme d'un gel, et
dans laquelle la composition ne comprend pas plus de 20 % en poids de solvants volatils.

2. Composition selon la revendication 1, présentant une viscosité de cisaillement, mesurée à température ambiante par rhéométrie rotationnelle à 100 1/s, de 0,1 à 10 Pa·s.

3. Composition selon l'une quelconque des revendications précédentes, dans laquelle le solvant hydrophile est glycérol, propylène glycol, éthanol, un polyéthylène glycol liquide, ou une combinaison de l'un quelconque de ceux-ci, éventuellement une combinaison de glycérol, de propylène glycol et d'éthanol.

4. Composition selon l'une quelconque des revendications précédentes, dans laquelle la quantité de solvant(s) hydrophile(s) dans la composition est comprise entre 65 % et 85 % en poids.

5. Composition selon l'une quelconque des revendications précédentes, dans laquelle la composition ne comprend pas plus de 15 % en poids de solvants volatils.

6. Composition selon l'une quelconque des revendications précédentes, dans laquelle l'agent augmentant la viscosité est un polymère, un copolymère ou un polymère réticulé à base de polyacrylate.

7. Composition selon la revendication 6, dans laquelle la quantité de polymère, de copolymère ou de polymère réticulé à base de polyacrylate dans la composition est comprise entre 0,1 % et 1 % en poids.

8. Composition selon la revendication 7, dans laquelle l'agent augmentant la viscosité est le polyacrylate crosspolymer-6.

9. Composition selon l'une quelconque des revendications précédentes, comprenant en outre du dexpanthénol.

10. Composition selon la revendication 9, dans laquelle la quantité de dexpanthénol dans la composition est comprise entre 1 % et 5 % en poids.

11. Composition selon l'une quelconque des revendications précédentes, essentiellement exempte de lipides ou d'huiles.

12. Composition selon l'une quelconque des revendications précédentes, dans laquelle la composition ne se présente pas sous la forme d'un vernis à ongles.

13. Tube métallique pour pommade comprenant la composition selon la revendication 1, dans lequel la composition comprend éventuellement :
- 0,25 % en poids à 1 % en poids de piroctone olamine ;
- 10 % en poids à 20 % en poids d'urée ;
- de 40 % en poids à 65 % en poids de glycérol ;
- de 10 % en poids à 25 % en poids de propylène glycol ;
- de 5 % en poids à 15 % en poids d'éthanol ;
- de 0,25 % en poids à 1 % en poids de polyacrylate crosspolymer-6 ; et
- jusqu'à 10 % en poids d'un ou plusieurs autres ingrédients actifs ou inactifs ;
et dans laquelle, en outre, éventuellement, la composition est essentiellement constituée de :
- 0,25 % à 1 % en poids de piroctone olamine ;
- 10 % à 20 % en poids d'urée ;
- de 1 % en poids à 3 % en poids de dexpanthénol ;
- de 40 % en poids à 65 % en poids de glycérol ;
- de 10 % en poids à 25 % en poids de propylène glycol ;
- de 5 % en poids à 15 % en poids d'éthanol ;
- de 0,25 % à 1 % en poids de polyacrylate crosspolymer-6.

14. Composition telle que définie dans l'une quelconque des revendications précédentes, destinée à être utilisée dans la prévention ou le traitement d'une infection fongique superficielle ou d'une affection liée à une infection fongique superficielle, dans laquelle, éventuellement, l'infection fongique superficielle est une onychomycose.
